# EUROPEAN PATENT SPECIFICATION

(11) **EP 0 448 856 B1**
(45) Date of publication and mention of the grant of the patent: **03.02.1993**
(21) Application number: 90303231.6
(22) Date of filing: 27.03.1990
(51) Int. Cl.: A61K 31/535, A61K 9/06

(54) **Ophthalmic preparations comprising timolol maleate**
Augenpräparate enthaltend timolol maleate
Préparations ophtalmiques comprenant du maléate de timolol

(43) Date of publication of application: 02.10.1991
(73) Proprietor: CHATFIELD PHARMACEUTICALS LIMITED, London SW5 9JL (GB)
(72) Inventor: Solomon, Montague Cecil, London SW3 (GB)
(74) Representative: SERJEANTS

(56) References cited:
- EP-A- 0 014 642
- GB-A- 1 256 502
- GB-A- 1 465 383
- GB-A- 1 524 405
- GB-A- 2 225 237

## Description

The invention relates to ophthalmic preparations for lowering intra-ocular pressure which is a cause of glaucoma in human beings and animals.

Patent Specification GB-A-1524405 relates to ophthalmic compositions comprising 1-t-butylamino-3-(4-morpholino-1,2,5-thiadiazol-3-yloxy)-2-propanol hydrogen maleate (timolol maleate) together with an ophthalmic carrier in the form of a solid, a vegetable oil, or a buffered isotonic liquid, or in which the carrier is a solid water-soluble polymer. In practice these compositions always contain buffering agents and preservatives. Suitable water-soluble buffering agents are alkali metal and alkaline-earth metal carbonates, phosphates, bicarbonates, citrates and borates, such as sodium phosphate, citrate, borate, acetate, bicarbonate and carbonate. These agents may be present in amounts sufficient to obtain a pH of the system of between 5.5 to 8.0. The suitable water-soluble preservatives that may be included are typically sodium bisulphate, sodium thiosulphate, ascorbate, benzalkonium chloride, chloro-butanol, thimerosal, phenylmercurate acetate, phenylmercuric borate, parabens, benzyl alcohol and phenyl ethanol. These agents may be present in amounts of from 0.001% to as much as 5% by weight.

The compositions are in multi-dose form. All ophthalmic preparations, whether drops, lotions or ointments, are required to be sterile; the purpose of the preservatives, which are typically benzalkonium chloride (0.01%) or phenylmercuric nitrate (0.002%) is to maintain sterility after the sealed lid or sealed screw cap of the multi-dose container has been breached. Contamination of the multi-dose container occurs easily, due both to the entry of atmospheric micro-organisms when the bottle is opened, and more typically due to contamination of the eye-dropper which invariably touches the eye surface or eyelids during application of the drops, such surfaces being typically non-sterile. The contaminated eye-dropper, which is attached to the screw cap, is then re-inserted into the multi-dose container for closure. In an attempt to avoid the growth of these contaminating micro-organisms, a high concentration of preservatives must be used.

The preservatives in use in ophthalmic solutions are powerful disinfectants, typically quaternary ammonium disinfectants such as benzalkonium chloride which may produce hypersensitivity after repeated applications and is also occasionally irritant. Additionally, since tear secretions drain into the nasal cavity, small amounts of the composition are generally absorbed into the systemic circulation from the conjunctival vessels or from the nasal mucosa. Ophthalmic solution entering the nasal cavity may itself then drain into the mouth and will eventually be absorbed through the buccal mucosa or swallowed and absorbed through the gastric mucosa.

Because the treatment of raised intra-ocular pressure needs long-term repeated administration the patient is exposed to the risk of adverse local or systemic effects from the preservatives, a situation which is clearly undesirable.

The invention provides an ophthalmic preparation comprising an aqueous or saline-aqueous solution of timolol maleate in uni-dose form in the absence of any preservative or buffering agent. The preparation may include sodium chloride or another reagent to increase the tonicity of the solution or to make it isotonic.

The method of preparation is simple and uncomplicated, requiring dissolution of timolol maleate and sodium chloride in water for injection or some similar sterile pyrogen-free water, and adjusting where necessary the pH to 7.0 (with limits of 6.5 - 7.5) with sodium hydroxide solution or some similar acceptable reagent, making to volume filtering and filling.

The preparation may be supplied in a plastics container, preferably of polypropylene or polyethylene, for topical application as single dose eye drops. The containers may be formed into a sheet by the blow-fill-seal process using a Rommelag (Trade Mark) or similar machine to make up a pack comprising a number of unit doses. The machine comprises a mould which is fed with thermoplastics material in tubular or granular form. The mould is closed, and a special mandrel is introduced to form a container by blowing. A metered amount of product solution, for example 0.1 to 0.3 ml, preferably 0.2 ml, is forced into the container. The mandrel is retracted, and sealing jaws close the container. A vacuum is formed, so the container is hermetically sealed. The sheet may be marked with dosage, lot number, expiry date and/or other information. A sheet or strip comprising say ten unit doses assists patient compliance.

Since the uni-dose container is filled and hermetically sealed under sterile conditions and remains so until the moment before use, the sterility of the ophthalmic solution when delivered to the patient, is assured. The undesirable preservatives, with the consequent potential risk of systemic or topical adverse effects, can therefore be safely omitted. Similarly, because the pH of the solution is adjusted by an acceptable simple reagent such as sodium hydroxide, the buffering agents may also be omitted with confidence that because the pH is at neutral and therefore physiological levels, no undue stinging, smarting or irritation of the eye will occur.

After instillation into the eye (or eyes) of the prescribed one or two drops the uni-dose container with any remaining unused ophthalmic solution should be discarded. Typically, only a small amount of ophthalmic solution should be present in each uni-dose container so that any remaining unused portion is not so great as to tempt the patient to retain the remaining unused portion for use at a later date with all the risks of bacterial contamination in the meantime. A unit dose of from 0.001 to 5.0 mg, and preferably from 0.005 to 2.0 mg, and especially from 0.005 to 1.0 mg of timolol maleate in a sterile preservative-free and buffer-free solution is generally applied to the human eye for the treatment of reducing raised intra-ocular pressure.

### Example 1

17.10g timolol maleate base and 17.50 g sodium chloride are dissolved in 4800 ml purified water. The pH is adjusted to 7.0 (∓0.1) by addition of 0.1 M sodium hydroxide. The final volume is adjusted to 5000 ml by adding purified water at 20°C. The solution is sterilized by filtration, and kept protected from light. The tonicity of the solution is physiological or near-physiological, being approximately isotonic with normal saline, and has an active ingredient content of 2.5 mg per ml, 0.25% w/v. It is fed into polypropylene uni-dose containers with a fill volume of 0.2 ml.

### Example 2

34.20 g timolol maleate base and 35.00 g sodium chloride are dissolved in 4800 ml purified water. The pH is adjusted to 7.0 (∓0.1) by addition of 0.1 M sodiumhydroxide. The final volume is adjusted to 5000 ml by adding purified water at 20°C. The solution is sterilized by filtration, and kept protected from light. The tonicity of the solution is physiological or near-physiological, being approximately isotonic with normal saline, and has an active ingredient content of 5.0 mg per ml, 0.5% w/v. It is fed into polypropylene uni-dose containers with a fill volume of 0.2 ml.

## Claims

1. An ophthalmic composition comprising an aqueous solution of timolol maleate characterised in that the composition is in uni-dose form and is free from any buffering agent or preservative.

2. A composition according to claim 1 characterised in that it further includes sodium chloride to increase the tonicity of the solution or to make it isotonic.

3. A composition according to claim 1 or claim 2 characterised in that the pH of the timolol maleate aqueous solution is adjusted to from 6.5 to 7.5 by the addition of sodium hydroxide.

4. A composition according to any preceding claim characterised in that it is supplied in polypropylene or polyethylene uni-dose containers which are formed by the blow-fill-seal process using a Rommelag or like machine filled under sterile conditions and hermetically sealed at the moment of filling.

5. A composition according to claim 4 characterised in that each container contains from 0.1 to 0.3 ml of the solution.

6. A composition according to any preceding claim characterised in that it contains from 0.001 to 5.0 mg of timolol maleate.

## Patentansprüche

1. Ophthalmische Zusammensetzung mit einem Gehalt an einer wäßrigen Lösung an Timololmaleat, dadurch gekennzeichnet, daß die Zusammensetzung in Einzeldosisform vorliegt und frei von jeglichen Pufferungsmitteln oder Konservierungsmitteln ist.

2. Zusammensetzung nach Anspruch 1, dadurch gekennzeichnet, daß sie zusätzlich Natriumchlorid umfaßt, um die tonische Beschaffenheit der Lösung zu erhöhen oder um sie isotonisch zu machen.

3. Zusammensetzung nach Anspruch 1 oder 2, dadurch gekennzeichnet, daß der pH-Wert der wäßrigen Lösung von Timololmaleat durch Zugabe von Natriumhydroxid auf 6,5 bis 7,5 eingestellt wird.

4. Zusammensetzung nach einem der vorstehenden Ansprüche, dadurch gekennzeichnet, daß sie in Einzeldosisbehälter aus Polypropylen oder Polyethylen bereitgestellt wird, die nach dem Blasfüll-Siegelungsverfahren unter Verwendung einer Rommelag-Vorrichtung oder einer ähnlichen Vorrichtung hergestellt, unter sterilen Bedingungen gefüllt und zum Zeitpunkt der Füllung hermetisch verschlossen werden.

5. Zusammensetzung nach Anspruch 4, dadurch gekennzeichnet, daß jeder Behälter 0,1 bis 0,3 ml der Lösung enthält.

6. Zusammensetzung nach einem der vorstehenden Ansprüche, dadurch gekennzeichnet, daß sie 0,001 bis 5,0 mg Timololmaleat enthält.

## Revendications

1. Une composition ophtalmique comprenant une solution aqueuse de maléate de timolol caractérisée en ce que la composition est sous forme d'unidoses et ne contient ni agent tampon ni agent préservatif.

2. Une composition conformément à la revendication 1 caractérisée en ce qu'elle comprend aussi du chlorure de sodium pour augmenter la tonicité de la solution et pour la rendre isotonique.

3. Une composition conformément à la revendication 1 ou la revendication 2 caractérisée en ce que le pH de la solution aqueuse de maléate de timolol est ajusté de 6,5 à 7,5 par l'ajout d'hydrate de soude.

4. Une composition conformément à toute revendication précédente caractérisée en ce qu'elle est fournie dans des boîtes d'unidoses en polypropylène ou en polyéthylène qui sont formées par le procédé souffler-remplir-sceller avec une machine Rommelag ou semblable remplie dans des conditions stériles et fermée hermétiquement au moment du remplissage.

5. Une composition conformément à la revendication 4 caractérisée en ce que chaque unidose contient de 0,1 à 0,3 ml de solution.

6. Une composition conformément à toute revendication précédente caractérisée en ce qu'elle contient de 0,001 à 5,0 mg de maléate de timolol.
